Europäisches Patentamt

European Patent Office

Office européen des brevets

(11) Publication number: 0 628 817 A1

# EUROPEAN PATENT APPLICATION

(21) Application number: 93201672.8

(22) Date of filing: 10.06.93

(51) Int. Cl.5: G01N 33/53, C12Q 1/68, C12P 21/08

(43) Date of publication of application:
14.12.94 Bulletin 94/50

(84) Designated Contracting States:
AT BE CH DE DK ES FR GB IE IT LI NL SE

(71) Applicant: Nederlandse Organisatie voor
toegepast-natuurwetenschappelijk
onderzoek TNO
Juliana van Stolberglaan 148
NL-2595 CL The Hague (NL)

(72) Inventor: van der Schans, Govert Paulus
Hoofdgroep Gezondheidsonderzoek TNO
Med.Bio.Lab.
Postbus 45 NL-2280 AA Rijswijk (NL)
Inventor: Timmerman, Arie Jacob
Hoofdgroep Gezondheidsonderzoek TNO
Med.Bio.Lab.
Postbus 45 NL-2280 AA Rijswijk (NL)
Inventor: Mars-Groenendijk, Rosalia
Hermanna
Hoofdgroep Gezondheidsonderzoek TNO
Med.Bio.Lab.
Postbus 45 NL-2280 AA Rijswijk (NL)
Inventor: van den Berg, Paula Theresia Maria
Hoofdgroep Gezondheidsonderzoek TNO
Med.Bio.Lab.
Postbus 45 NL-2280 AA Rijswijk (NL)

(74) Representative: Smulders, Theodorus A.H.J.,
Ir. et al
Vereenigde Octrooibureaux
Nieuwe Parklaan 97
NL-2587 BN 's-Gravenhage (NL)

(54) Method for detecting single-strand breaks in DNA.

(57) Detection of single-strand breaks in cellular DNA by incubating a sample comprising cells containing said DNA with an alkaline medium capable of releasing DNA from the cells and at least partially unwinding said DNA, and subjecting the alkaline medium-treated sample to a sandwich immunoassay using a monoclonal antibody directed against single-stranded DNA to determine the amount of single-stranded DNA therein. The method is particularly suited for estimating the quality of sperm of vertebrates. To allow a more accurate estimation of the number of single-strand breaks in the DNA, aliquots of the sample may be subjected to a pretreatment that induces single-strand breaks, such as irradiation with γ- or X-rays, using various doses to allow making a dose-effect curve.

FIG. 4

FIELD OF THE INVENTION

The invention relates to a test method for detecting single-strand breaks in DNA as contained in cells (cellular DNA), in particular sperm DNA in order to determine the sperm quality.

BACKGROUND OF THE INVENTION

The upkeep and improvement of the Dutch stock of cattle occurs almost exclusively by artificial insemination. The presence of the correct combination of genetic properties makes the sperm of a selected sire very valuable. Sperm quality decreases without doubt during freezing of the sperm in small portions. In addition, it appears that sperm-DNA contains large amounts of oxidative damage, possibly due to the absence of DNA repair and as a result of deficient feeding, or aging of the sperm in the animal.

Sensitive immunochemical methods to analyse strand breaks (including alkali-labile sites) in DNA of sperm may be very useful to control sperm quality and to improve freeze-thawing procedures of the sperm. The methods may be valuable for the prediction of fertilization efficiency of the sperm. Also in pig- and horse-farming this method may be of value. Similarly, it may be of value to have a means to estimate the quality of the sperm of price-winning male mammals such as dogs and cats, or even male vertebrates such as pigeons and roosters. There may be also possibilities for application of the above-mentioned assays with regard to quality control of human sperm to be used for *in vitro* fertilization.

After oocyte penetration the paternal contribution to the development potential of the conceptus depends entirely on the quality of the spermatozoon that penetrated, *i.e.* on the intrinsic sperm quality. Studies on sperm cell characteristics related to intrinsic sperm quality such as the degree of DNA condensation and the amount of DNA damage are relatively new and their predictive value for the probability of a successful gestation is not yet fully established.

Evenson (1986) described flow cytometry of acridine orange-stained sperm as being a rapid and practical method for monitoring occupational exposure to genotoxicants. Evenson and Thompson (1992), applying this method, reported about a good correlation between the number of cells scored outside the main population in the flow cytogram and the decrease in fertility of 6 different boars. However, from this study it is not clear whether the deviation from the normal population is due to DNA damage or due to other changes.

Fraga *et al.* (1991) demonstrated that human sperm DNA contains large amounts of oxidative DNA damage. They applied a rather laborious method for isolation and enzymatic digestion of DNA followed by analysis by HPLC with electrochemical detection of $oxo^8dG$ (8-hydroxy-2'-deoxyguanosine). Due to the high level of this type of damage in these cells they were able to demonstrate a decrease in this level after dietary application of ascorbic acid. The reason for these high levels of (oxidative) damage may be the absence of any DNA repair in spermatozoa.

In previous experiments we could show that already in a precursor stage of the spermatogenesis in the hamster (*i.e.* in the elongated spermatid-stage) there is no repair of ionizing radiation-induced single-strand breaks (Van Loon *et al.*, 1991a). The observed high levels of damage in DNA of spermatozoa may, therefore, be the result of an accumulation of unrepaired oxidative damage, induced spontaneously or as the result of normal metabolic processes.

Several methods are currently used to quantify damage in DNA of mammalian cells, *viz.* the alkaline sucrose-gradient centrifugation (McGrath and Williams, 1966; Van der Schans *et al.*, 1982), the alkaline unwinding (Ahnström and Erixon, 1973; Rydberg, 1975, 1980), the alkaline elution (Kohn and Grimeg-Ewig, 1973; Van Loon *et al.*, 1991b), the alkaline pulse field gel electrophoresis (Sutherland *et al.*, 1987), the competitive enzyme-linked immunosorbent assay (ELISA; Van der Schans *et al.*, 1989) and the comet assay (Olive *et al.*, 1990).

As discussed extensively in our previous publications (Van der Schans *et al.*, 1989, Van Loon *et al.*, 1992), these methods, however, have one or more of the following drawbacks:

(i) large numbers of cells are required [alkaline unwinding: $5\text{-}10 \times 10^6$; alkaline elution: $2\text{-}4 \times 10^6$],

(ii) cellular DNA must be radiolabelled (which will not allow analysis of damage in DNA of non-cycling cells and precludes *in vivo* experiments) [some versions of the alkaline unwinding and alkaline elution],

(iii) the technique is laborious and rather time-consuming [alkaline unwinding: cells need to be isolated and, after treatment with alkali, each sample needs to be separated over a hydroxyapatite column and counted for radioactivity; alkaline elution: elution takes place overnight, measurement of radioactivity or fluorescence in each fraction; pulse field electrophoresis: runs take place overnight], or

(iv) require complicated image analysers and computer systems to measure the distribution of DNA in the gels to interpret the results [comet assay and pulse field electrophoresis].

Recently, an assay to determine radiation-induced single-strand breaks has been based on the application of monoclonal antibodies directed specifically against single-stranded DNA (Van der Schans *et al.*, 1989, Van Loon *et al.*, 1992). Such antibodies have also been described by Ballard *et al.*, 1984, Ballard and Voss, 1985, Frankfurt, 1987, Fukada *et al.*, 1988 and Traincard *et al.*, 1989, but these have not been used for strand break detection.

The assay described by Van der Schans *et al.*, 1989, and Van Loon *et al.*, 1992, is a combination of the so-called unwinding assay with an immunochemical quantification of the single-stranded DNA formed during the unwinding. The essence of the method is that the limited local single-strandedness associated with each (double- and single-) strand break (and with each lesion converted into such breaks in alkaline medium) is converted - by strictly controlled partial unwinding in alkaline medium - into a long stretch of single-stranded DNA to which the antibody molecules can bind. This unwinding step has resulted in a substantial increase in the sensitivity of the immunochemical detection of single- and double-strand breaks. Since each breakage site forms an initiation point for the unwinding process, the extent of unwinding (% single-strandedness) is a measure of the number of such sites.

The assay does not require prelabelling of the cells with radioactive precursors of DNA and is therefore applicable to non-dividing cells and to DNA obtained from *in vivo* experiments.

In the assay developed by Van der Schans *et al.*, 1989, the monoclonal antibodies are used in a competitive ELISA (enzyme-linked immunosorbent assay) with the partially unwound DNA used as the competitor. It was shown to be sufficiently sensitive to determine the breaks induced by 0.5 Gy of γ-rays.

Van Loon *et al.* (1992) describe a modification of this assay in which the DNA to be tested is directly adsorbed to the wells of a microtiter plate. The latter method was used to quantify the amount of DNA damage in human white blood cells after *in vitro* exposure to ionizing radiation. The assay has about the same sensitivity as the competitive ELISA (Van der Schans *et al.*, 1989) but requires only half the amount of blood, *i.e.*, about 50 μl. Moreover, about 30% fewer handlings are needed and the time required after collection of the blood to obtain the final result has been reduced from 5 to 3.5 h, which might be important for some applications.

SUMMARY OF THE INVENTION

An object of the invention is to provide a method for detecting single-strand breaks in cellular DNA which method is applicable to non-purified complex mixtures such as blood or sperm, can be carried out in a relatively short time and gives an accurate estimation of the number of single-strand breaks in the DNA.

The invention provides such a method for detecting single-strand breaks in cellular DNA, comprising the steps of incubating a sample comprising cells containing said DNA with an alkaline medium capable of releasing DNA from the cells and at least partially unwinding said DNA, and subjecting the alkaline medium-treated sample to a sandwich immunoassay using a monoclonal antibody directed against single-stranded DNA to determine the amount of single-stranded DNA therein. Said monoclonal antibody preferably is D1B, ECACC No. 93021026.

In a particularly preferred embodiment of the subject invention, said sample consists of sperm of a vertebrate, more preferably sperm of a mammal (this term including man).

In another particularly preferred embodiment of the invention, the sample is subjected to a treatment capable of inducing single-strand breaks in the cellular DNA before the treated sample is incubated with the alkaline medium.

A highly preferred method of the invention comprises *i*) irradiation of sperm samples with various doses of γ-rays (or X-rays), *ii*) release of DNA from the sperm immediately followed by controlled unwinding of the DNA by a treatment with alkali, and, as the extent of unwinding is related to the amount of oxidative damage in the DNA, such as single-strand breaks, *iii*) determination of the amount of single-stranded DNA in the generated mixture with a newly developed sandwich immunoassay. The technique thus comprises the determination of the percentage of single-strandedness resulting from the partial unwinding of cellular DNA under strictly controlled alkaline conditions. The amount of oxidative damage in non-irradiated sperm is expressed as the radiation dose required to double the measured amount of single-strandedness.

DETAILED DESCRIPTION OF THE INVENTION

The invention provides a sensitive, simple and rapid immunochemical method for the detection of single-strand breaks in cellular DNA with particular utility for detecting single-strand breaks in sperm DNA of vertebrate origin as a means to estimate the quality of the sperm.

Advantages of the method are that:

1) no radioactive labelling of cellular DNA is required,

2) when applied on complex materials such as human blood or sperm, the sample does not require any (pre)purification,

3) damage can be detected after radiation doses as low as 0.2 Gy, corresponding to about 200 single-strand DNA breaks per cell,

4) the assay takes only 1.5 h after the collection of blood or any other sample,

5) the assay can be applied on several other types of cells that can be obtained in suspension.

The invention provides procedures and particular conditions therein, including the conditions required for the DNA release and the controlled unwinding of DNA as well as a newly developed sandwich immunoassay procedure, in particular for a sandwich ELISA. The prior art does not teach a sandwich immunoassay for the detection of damage (single-strand and double-strand breaks and alkali labile sites) in DNA of white blood cells or other types of cells exposed to biologically relevant doses of ionizing radiation or other genotoxic physical and chemical agents.

With the aim to develop a sensitive, quantitative detection of the degree of alkali-enhanced single-strandedness in cellular DNA, we adapted a standard immunological assay procedure, the sandwich ELISA (Campbell, 1984). This method is based on the binding of antigen (in our case single-stranded DNA) to antibody molecules that are covalently bound to the wells of plastic microtiter plates. Binding of this antigen is assayed via adherence of the same type of antibodies carrying an enzyme, the activity of which can be tested via incubation with a colorigenic or fluorigenic substrate.

In the final procedure that we developed, aliquots from DNA samples composed of a mixture of double- and single-stranded fragments are pipetted into the wells of, *e.g.*, a polystyrene microtiter plate which are coated with monoclonal antibodies specific for single-stranded DNA (preferably the monoclonal antibody D1B). Additionally, a second aliquot of each DNA sample is heated or subjected to a second treatment with alkali to make the DNA completely single-stranded, and then brought into a well. Only the single-stranded form of DNA binds efficiently to the immobilized antibodies in the well. The amounts of single-stranded DNA attached to the wells are determined using the same antibodies but now carrying a conjugated enzyme (or any other suitable marker).

Assays involving the binding of antigen to immobilized antibodies are widely applied nowadays. Generally, however, the antibodies carrying the detection enzyme must be directed against other epitopes of the antigen molecule than the antibodies applied for the coating (*i.e.* used for the immobilization of the single-stranded DNA). In the sandwich ELISA presented herein this appeared to be unnecessary. Each piece of single-stranded DNA contains several sites specific for the D1B antibodies of which only a small fraction is occupied by the binding of the fragment to the antibodies on the wall.

The advantages of direct attachment of single-stranded DNA from a mixture of single-stranded and double-stranded DNA to D1B-coated polystyrene compared to the direct attachment of single- and double-stranded DNA to polystyrene (Van Loon *et al.*, 1992) are the gain in time and the greater simplicity. In the assay of Van Loon *et al.* (1992), after the DNA adsorption the wells have to be treated with fetal calf serum (FCS) to prevent non-specific binding of antibody in the subsequent detection step; a great advantage of the sandwich ELISA is that the single-stranded DNA pieces are very specifically bound to the antibodies on the wall in spite of the presence of large amounts of double-stranded DNA, RNA, proteins and any other substances present in complete blood. Treatment with FCS is also required in the sandwich ELISA, but in this case, it can be carried out before DNA binding takes place. Therefore, this time-consuming step is not a part of the assay itself.

While in the assay of Van Loon *et al.* (1992) the white blood cells first have to be carefully isolated since several blood components interfere with the passive binding of DNA to the wall, this is not necessary in our sandwich immunoassay. This is an important advantage of our method particularly for *in vivo* studies: when the amount of DNA damage induced by ionizing radiation or any other DNA damaging agent can be determined without purification of the cells, fewer cells are needed because loss of cells during purification is avoided.

The immunochemical method according to the invention differs from the previously proposed immunoassays in that only the single-stranded DNA in the sample to be tested becomes attached to the wells of a microtiter plate, owing to the fact that the wells are coated with monoclonal antibodies directed against single-stranded DNA. The amount of DNA bound is then quantified via the binding of the same type of monoclonal antibody (i.e. also directed against single-stranded DNA), but now conjugated with an assayable enzyme. By comparing this amount with the quantity of DNA measured after total unwinding the degree of single-strandedness can be established which is a measure of the DNA damage present in the sample before the alkaline treatment.

4

Application of antibodies directed against single-stranded DNA to determine the amount of single-stranded DNA in the way indicated above has never been described. In addition, the invention allows to use conjugates of the same antibody with alkaline phosphatase or another detectable enzyme or any other detectable label (fluorescing probes, isotope labels, etc.) for detecting the antibody-bound DNA in the sandwich immunoassay. Of course, it is also possible to use different monoclonal antibodies directed against single-stranded DNA, if different monoclonals are available.

In a preferred embodiment of the invention, at least one of said monoclonal antibodies is D1B, ECACC No. 93021026.

The monoclonal antibody D1B which is directed against single-stranded DNA, and conjugates of said antibody with the enzyme alkaline phosphatase (or any other detectable marker) have not been available to the public.

The terminology "directed against single-stranded DNA" as used herein is meant to indicate a specific affinity for single-stranded DNA and absence of any significant affinity for double-stranded DNA, owing to which the antibody will bind to single-stranded DNA, if present, and will essentially not bind to double-stranded DNA.

The method described herein for the DNA release from a complex material such as sperm (of vertebrates or preferably mammals) immediately followed by a controlled unwinding of the DNA is a newly developed method not known before.

A fact is that the extent of DNA unwinding depends on several factors, such as small differences in pH, temperature and buffer capacity of the sperm. Further, the fluorescence of a completely denatured sample cannot be assessed with high accuracy in practice. These uncertainties can be circumvented by irradiating the sperm sample, determining a dose-effect curve and expressing the amount of DNA damage in the non-irradiated samples as Gray-equivalents. Instead of irradiating the sample, also other treatments capable of inducing single-strand breaks in the cellular DNA may be used, *e.g.* treatment with a chemical agent causing single-strand breaks in the cellular DNA. Preferably, however, the treatment capable of inducing single-strand breaks in the DNA comprises irradiating the sample, such as with $\gamma$-rays or X-rays. The radiation dose will usually vary from about 0.2 Gy to about 2000 Gy.

With an appropriate choice of the ionic strength of the lysis and unwinding buffers the application range of the procedure can be substantially extended, corresponding to a radiation dose of 0.2 Gy - 2000 Gy and the procedure can be applied on several types of cells.

In particular in the case of a sperm sample, it is preferred that the alkaline medium capable of releasing DNA from the cells and of at least partially unwinding the DNA has a pH of above about 12 and comprises alkali metal hydroxyde, urea, dithiothreitol and a surfactant. The alkaline medium preferably has a pH of from about 12.05 to about 12.25, and the surfactant preferably comprises a non-ionic surfactant, such as Triton X100 in particular. Other surfactants (such as Tween 20) may be suitable also, but are less preferred than Triton X100. In a particularly preferred embodiment of the invention, the alkaline medium comprises about 0.4-1.0 M urea, about 40-45 mM NaOH, about 14-18 mM dithiothreitol and about 0.03-0.1% Triton X100 (hereafter referred to as solution A).

The incubation with alkaline medium is preferably carried out for about 3-15 minutes, *e.g.* for about 7 minutes.

Thereafter, the alkaline medium-treated sample is preferably neutralized and sonicated before it is subjected to the sandwich immunoassay. The neutralization and sonication conditions are not critical and may be essentially identical to those described in the cited articles of Van der Schans *et al.*, 1989, and Van Loon *et al.*, 1992. As described therein, an aqueous solution of $NaH_2PO_4$ (*e.g.* 250 mM) may be used for said neutralization, and sonication (to fragment the DNA in order to prevent rewinding) may be effected in a few seconds (*e.g.* about 10 seconds) with suitable sonication apparatus such as Ultrasonics W-370, U.S.A., with microtip, output level 2.5.

The results indicate that after alkali treatment in the presence of dithiothreitol, urea and Triton X100, radiation-induced breaks in sperm DNA can be detected besides eventually already present oxidative damage. Remarkably, frozen sperm contains, after thawing, already a very large amount of single-strand breaks (corresponding to 100-600 Gy $\gamma$-rays) which differs per sire. With human sperm analogous conclusions can be drawn. The amount of damage ($10^5$ lesions per cell) is of the same order of magnitude as reported for 8-OHdG (Fraga *et al.*, 1991). This means that we have developed a method to detect and quantify oxidative damage in sperm DNA. This opens the way for numerous studies with respect to maintenance and quality of sperm of different sires, donors etc.

In conclusion, a simple, sensitive and fast immunochemical method has been developed to quantify the amount of DNA damage that can be detected as strand breaks in human blood after *in vitro* exposure to ionizing radiation. The assay takes only 1.5 h after the collection of blood to yield an answer about the DNA damage induced. Damage can be detected after doses as low as 0.5 Gy. In addition, only 15 $\mu$l of blood is required per assay, without the need for a radioactive labelling of the DNA and without any prepurification of cells or DNA. In principle, single-strandedness can be detected in any (mammalian or vertebrate) cell type that can be prepared as a homogeneous suspension (as with the direct ELISA described previously) provided the DNA is released under the mild alkaline treatment applied. Therefore, this assay may have applications in several areas related to human health.

It is noted that the invention also allows detection of more persistent damages in DNA by starting with conversion of persistent base damage into single-strand breaks or alkali-labile sites. This conversion can be induced by treatment of permeabilized cells with damage-specific glycosylases or damage-recognizing endonucleases (*e.g.* those present in *Micrococcus luteus* extracts).

## BRIEF DESCRIPTION OF THE DRAWINGS

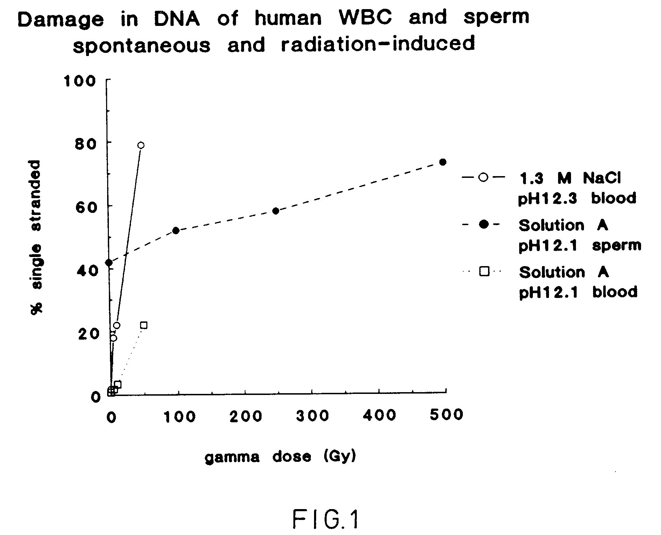

Fig. 1. Damage in DNA of $\gamma$-irradiated human sperm and in DNA of white blood cells in $\gamma$-irradiated human blood, assayed under different alkali-treatment conditions. Sperm treated with solution A of pH 12.1: (closed circles); WBC treated with solution A of pH 12.1: (open squares); WBC treated with an 1.3 M NaCl solution of pH 12.3: (open circles).

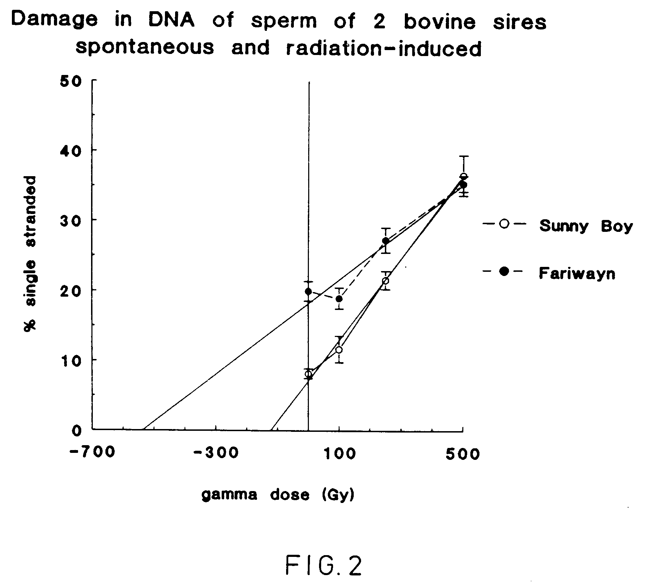

Fig. 2. Oxidative damage and radiation damage after *in vitro* irradiation of frozen and thawed bovine sperm of two sires. Sperm of sire A appeared to be more fertile than that of sire B. Sire A: (open circles); Sire B: (closed circles).

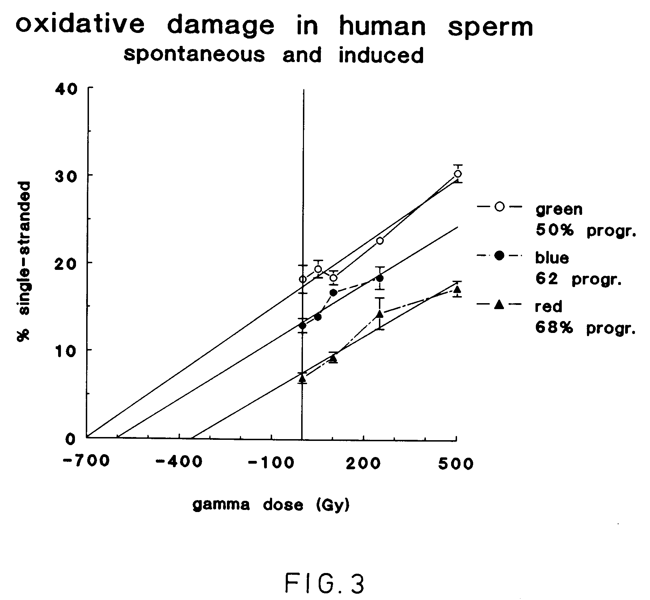

Fig. 3. Oxidative damage and radiation damage after *in vitro* irradiation of frozen and thawed human sperm of three donors. The progressive motility of the samples was: 50% (open circles); 62% (closed circles); and 68% (closed triangles).

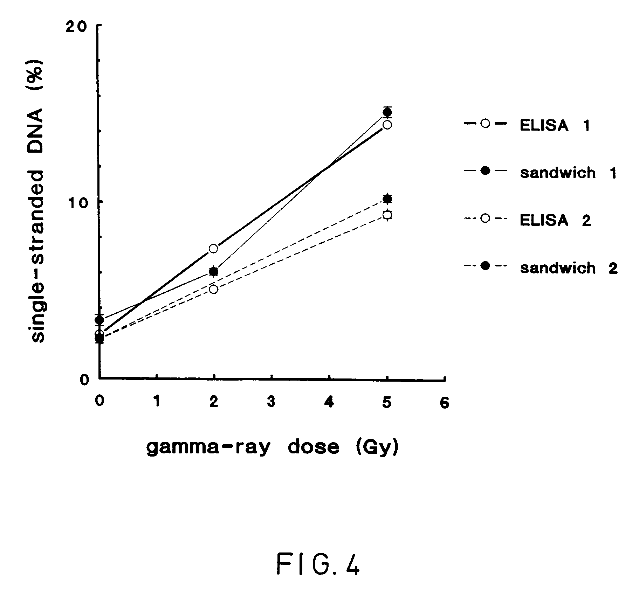

Fig. 4: Comparison of the sandwich ELISA with the previously applied (indirect) ELISA. Radiation damage in DNA that gives rise to single-strandedness in alkali assayed as a function of radiation dose of human blood exposed to ionizing radiation. After *in vitro* irradiation of human whole blood with $^{60}$Co-$\gamma$-rays the white cells were isolated, treated with alkali (16 min at 20°C) followed by analysis of the degree of single-strandedness with the ELISA or the sandwich ELISA. Two completely different series of experiments are shown of which the conditions of unwinding of the DNA were slightly different with respect to pH. The broken lines correspond to the one, the straight lines to the other series. The closed symbols represent data obtained with the sandwich ELISA and the open symbols those obtained with the normal ELISA. The values presented are the averages of duplicate determinations.

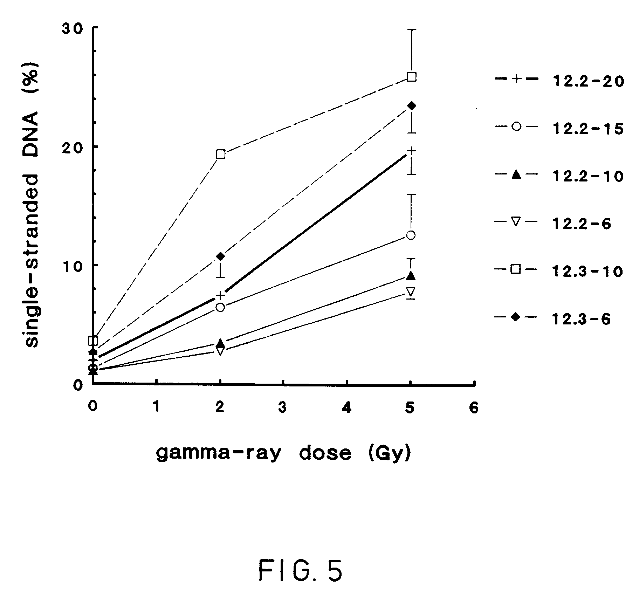

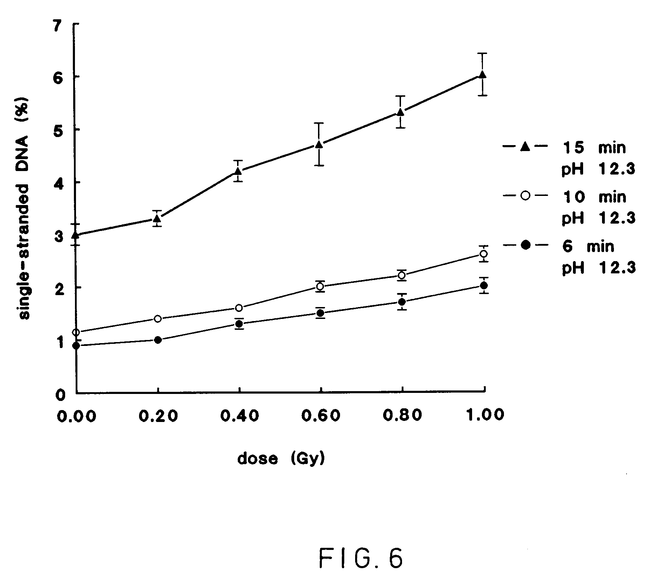

Fig. 5: Radiation damage in DNA that gives rise to single-strandedness in alkali, assayed under different alkali treatment conditions. After *in vitro* irradiation of human whole blood with $^{60}$Co-$\gamma$-rays, the white cells were isolated, treated with alkali (16 min at 20°C) followed by analysis of the degree of single-strandedness with the sandwich ELISA. The alkali-solutions and periods of treatment applied were 1.3 M NaCl adjusted with 1.0 M NaOH to pH 12.2 and incubated for 6 min (open triangles), 10 min (closed triangles), 15 min (open circles) or 20 min ("plus" signs) or adjusted to pH 12.3 and incubated for 6 min (closed diamonds) or 10 min (open squares). The values presented are the averages of duplicate determinations. The error bars represent the range of the data. Fig. 6. Radiation damage in DNA giving rise to single-strandedness in alkali, assayed at different alkali-treatment conditions after *in vitro* irradiation of whole human blood with low doses 300-kV X-rays. After irradiation, the blood was treated with alkali (pH 12.3) for: 15 minutes (closed triangles); 10 minutes (open circles); and 6 minutes (closed circles). The data are averages of two determinations. The error bars represent the range of the data points.

## EXAMPLES

### I Materials and methods

### *Procedure for the detection of oxidative damage in sperm DNA according to the invention*

The procedure can be divided into 3 steps.

1. Sperm (bovine or human) is divided over 4 tubes and then irradiated with various doses of $^{60}$Co-$\gamma$-rays (0-500 Gy).

2. Next, each sample is diluted 40x in PBS (phosphate buffered saline; 0.14 M NaCl, 8.1 mM $Na_2HPO_4$, 15 mM $KH_2PO_4$, 2.6 mM KCl, pH 7.4). To 60 $\mu$l of this suspension 415 $\mu$l of solution A is added which contains: 0.5 M Urea, 43 mM NaOH, 16 mM dithiothreitol and 0.06% Triton X100 (with a final pH of about 12.1). After 7 min at 20°C the mixture is neutralized with 100 $\mu$l 250 mM $NaH_2PO_4$ and sonicated

during about 1 sec.

3. In the mixture of single- and double-stranded fragments generated in this way, the amount of single-stranded DNA is determined by means of a sandwich ELISA. The sandwich-ELISA is based on the binding of the single-stranded DNA to an immobilized monoclonal antibody. The amount of bound single-stranded DNA is determined by addition of the same antibody conjugated to an assayable enzyme, e.g. alkaline phosphatase. The amount of substrate that can be converted into a (*e.g.*, fluorescent) product is a measure of the amount of bound single-stranded DNA. The total amount of DNA can be determined with the same system by first making the DNA completely single-stranded (by addition of alkali or by heating for 2 min at 100°C).

The fraction single-stranded DNA is a measure of the amount of oxidative damage in the non-irradiated sample (becoming manifest as single-strand breaks after alkali treatment) + the radiation-induced single-strand breaks.

### Sources of sperm

Frozen or fresh (unfrozen) bovine sperm was supplied in straws by Holland Genetics (Arnhem, The Netherlands). Sperm was diluted in nitrogenated buffer containing 199 mM Tris, 65 mM citric acid, 56 mM fructose, 5.6% glycerol (v/v) and 20% yolk of hen's egg (v/v), pH 6.8, to concentrations indicated in the text.

Frozen human sperm was supplied in straws by the Centre of In Vitro Fertilization, Academic Hospital of the Free University (Amsterdam, The Netherlands) at concentrations of 10-50 x $10^6$ cells/ml.

### Human white blood cells

Ten ml venous blood was collected from volunteers in evacuated 10-ml glass tubes containing 15 mg EDTA. Immediately after irradiation, the blood was either used directly or the white blood cells were isolated first. To this aim, 0.30 ml blood was mixed with 6 ml icecold lysis buffer (155 mM $NH_4Cl$, 10 mM $KHCO_3$, 0.1 mM $EDTA-Na_2$, pH 7.0) and kept at 0°C to prevent repair. As soon as lysis of the erythrocytes was complete (after about 4 min), the white blood cells were collected by centrifugation (10 min 4°C, 200 g at the tube bottom), washed twice in Hanks' balanced salt solution and resuspended (finally) in PBS to a final concentration of 1.6 x $10^6$ cells/ml.

### Irradiations

Sperm or human blood, collected as described, was irradiated at room temperature in 10-ml polystyrene test tubes, with a $^{60}Co$-γ-source (Gamma cell 200, Atomic Energy of Canada Ltd., Ottawa, Canada) at a dose rate of 20 Gy/min or a Philips 300-kV X-ray machine (at a dose rate of 4 Gy/min) with the characteristics: 300 kV, 10 mA, 1.5 mm Cu filter (corrected), hvl 2.9 mm Cu, average distance to the target 23 cm.

### Preparation of DNA samples of WBC for the sandwich ELISA

About $10^5$ white blood cells suspended in 60 μl PBS at 0°C were injected either into 415 μl 1.3 M NaCl that had been adjusted with the calculated amount of 1 M NaOH to pH 12.1 at 20°C, or into another solution if indicated. The addition of the cell suspension lowered the pH by about 0.15 unit (The pH was measured with a Radiometer (Copenhagen, Denmark) PHM82 pH meter equipped with a B-glass electrode (type GK2402B) at 20°C). The mixture was kept in the dark (red light) at 20°C. After 7 min the solution was neutralized with 100 μl 250 mM $NaH_2PO_4$, then the DNA was fragmented by sonication (Ultrasonics W-370, USA, with microtip, output level 2.5) for 5 sec to prevent rewinding. After this treatment the DNA consists of a mixture of double- and single-stranded fragments. The samples were stored at 4°C until they were used.

### Preparation of DNA samples of human blood for the sandwich ELISA

Blood (15 μl), collected as described, was brought into a roundbottom glass tube and diluted 4x with PBS. Then, 415 μl 1.3 M NaCl, adjusted with the calculated amount of 1 M NaOH to pH 12.3, was added at 20°C. The mixture was kept in the dark (red light) at 20°C and handled further as described in the previous section.

*Preparation of antibody directed against single-stranded DNA*

The monoclonal antibody D1B directed against single-stranded DNA was obtained as a by-product during the search for DNA-adduct specific antibodies, as described previously (Van der Schans *et al.*, 1989). Briefly, hybrid cells were obtained after immunization of mice with benzo(a)pyrene (BP)-diolepoxide-treated calf thymus DNA conjugated with methylated bovine serum albumin and subsequent fusion of the spleen cells with plasmacytoma cells. Specific antibody production was assayed in a direct ELISA with control DNA and BP-modified DNA as immobilized antigens. Several clones produced antibodies against control DNA without preference for BP-modified DNA. The antibodies of one of these, D1B, were found to specifically recognize single-stranded DNA. These antibodies were produced by culturing the hybrid cells in RPMI 1640 medium (Gibco, Breda, Netherlands), the supernatant of which was used in the ELISA (Van der Schans *et al.*, 1989; Van Loon *et al.*, 1992).

For the coating of microtiter plates and the preparation of D1B-alkaline phosphatase conjugates, the antibodies were purified by precipitation in ammonium sulfate at 50% saturation. The pellet was dissolved in PBS and further purified by chromatography through an Ultragel ACA 54 (Pharmacia, Uppsala, Sweden) column (85 x 1.6 cm) with PBS. The fractions with antibody activity (assayed in a direct ELISA) were pooled and concentrated in a dialysis bag embedded in dry polyethylene glycol 20,000 (taking about 7 h at 4°C) and finally dialysed against PBS. The protein concentration was determined by measuring the absorbance at 280 nm assuming an $A_{280}$ of 1.4 per mg protein. The yield of purified D1B antibody originating from 0.5 liter culture supernatant amounted to about 72 mg protein in a volume of 16 ml PBS. The antibody is of the IgM subclass.

The hybridoma clone D1B which produces said IgM-type monoclonal antibody D1B was deposited on 8 February 1993 in accordance with the terms of the Budapest Treaty 1977 with the European Collection of Animal Cell Cultures (ECACC), Porton Down, Salisbury, Wiltshire, United Kingdom, deposit number ECACC 93021026.

*Preparation of alkaline phosphatase-labelled D1B monoclonal antibody conjugates*

The conjugation with alkaline phosphatase (type VII-T, 11.1 mg/ml, 25,000 units, Sigma Chemical Co. St. Louis, MO, P-6774) was carried out essentially as described by Claassen and Adler (1988). Briefly, 10 mg alkaline phosphatase, diluted with 2 ml PBS, was dialysed extensively against 1 liter PBS at 4°C during 4 h and then against 100 ml 0.2% glutaraldehyde in PBS, at 4°C and overnight. Excess glutaraldehyde was removed by dialysis against PBS and the activated alkaline phosphatase was transferred to a test tube with 5 mg D1B (in 2 ml PBS). After overnight incubation at 4°C, 0.2 ml 0.2 M glycine.HCl was added, and after 2 h at room temperature this mixture was dialysed against PBS, diluted 1:2 with glycerol, and stored at -20°C in 0.5-ml aliquots, containing about 0.7 mg protein/ml. Before use, conjugates were diluted with PBS containing 5% FCS (fetal calf serum, Flow laboratories, Irvine, United Kingdom) and 0.05 % SDS (sodium dodecyl sulfate); they were used at 0.7 $\mu$g/ml.

*Preparation of D1B-coated microtiter plates*

The wells of high binding polystyrene microtiter plates (96-well; Catalog Number 3590, Costar Europe Ltd., Badhoevedorp, Netherlands) are filled with 100 $\mu$l purified D1B-antibodies (10 $\mu$g D1B/ml PBS) and incubated at room temperature under continuous vibration for 10 min. The wells are washed 3 times with PBS and then incubated with 150 $\mu$l of PBS + 5% FCS for 10 min at room temperature under vibration to minimize nonspecific DNA- and antibody-binding in subsequent steps. The wells are washed 3 times with PBS. When stored dry at -20°C, the plates retain their properties for at least several months.

*Procedure of the sandwich ELISA*

The 12 wells in the first row of a D1B-coated polystyrene microtiter plate (see above) are filled with 120 $\mu$l PBS, the other rows with 70 $\mu$l/well. Of each DNA sample (a mixture of single- and double-stranded fragments) a 20-$\mu$l aliquot is added to a well of the first row. Two further aliquots are pipetted into wells number 11 and 12, after a 10-fold dilution in PBS + 0.04% SDS and heating for 1.5 min in boiling water. Immediately after addition of the samples to the first row, twofold serial dilutions of these solutions are made in the following 6 rows, the last row (row H) is used to determine the background. In this way, 10 DNA samples can be assayed per plate. Then, the plate is left at room temperature for at least 5 min with continuous vibration on a Titertek vibration device (Flow Laboratories) which results in maximal adsorption (at least 70% at the appropriate dilutions) of the single-stranded DNA (checked by detection of DNA-binding after transfer of the remaining solution from the wells to those of another D1B-coated microtiter plate).

The wells are washed 3 times with PBS and then incubated with 100 $\mu$l of D1B-alkaline phosphatase conjugate, diluted 1:1000, in PBS + 0.05% SDS + 5% FCS for 5 min at room temperature again with continuous shaking. After repeated washing with PBS + 0.05% Tween 20 and once with 0.1 M diethanolamine (pH 9.8), 0.2 mM 4-methylumbelliferyl phosphate is added. After incubation for 1 h at 37°C, the fluorescence is recorded with a Fluoroskan (Eflab, Finland).

*Calculation of the percentage single-strandedness*

Provided limited amounts of DNA are used a linear relationship is obtained, for both methods, between the input amount of DNA and the level of fluorescence, both for completely and for partially single-stranded DNA. To calculate the fraction of single-strandedness in a particular sample, the ratio of the fluorescence of the DNA dilutions to that of the corresponding completely single-stranded DNA dilutions, both corrected for background fluorescence (fluorescence in wells without DNA), is divided by a factor of 10 (because of the predilution of the completely single-stranded DNA sample). In formula:

$$\% \text{ single-strandedness} = \frac{(fl_{sample} - fl_{background})}{10 \times (fl_{100\% \ ss} - fl_{background})} \times 100 \ \%$$

wherein
fl = fluorescence;
$fl_{background}$ = fluorescence in well without coating DNA;
100% ss = sample of which the DNA is completely single-stranded.

The calculations are carried out only for those dilutions for which a linear relationship is observed between the amount of DNA in the wells and the level of fluorescence. In daily practice, the percentage of single-strandedness calculated for the highest amounts of DNA in the well is used.

II Results

*Release of DNA*

In one experiment we applied a method for lysis of sperm cells with which we were successful with somatic cells in alkaline elution experiments (Van der Schans *et al.*, 1989). To this purpose, sperm was first diluted in Tris/EDTA buffer and treated with proteinase K + SDS (sodium dodecyl sulphate). In an alternative experiment, we used sperm diluted in Tris/EDTA and treated with proteinase K and Triton X100 and, in another experiment, sperm diluted in Tris/EDTA and treated with proteinase K without any detergent. We applied these methods on frozen and thawed bovine sperm. None of these experiments gave satisfying results. We never obtained complete release of DNA from the sperm heads upon addition of alkali.

We studied other alternatives to release the DNA from the sperm heads upon addition of alkali. It appeared possible to obtain a complete release of DNA by treatment of the sperm with 3 M Urea + 0.5 M $\beta$-mercaptoethanol (Balhorn *et al.* 1977). A disadvantage of this approach was, however, that simultaneous with the addition of the alkaline solution, the cells were destroyed, so the alkaline treatment itself induced a large amount of DNA-damage (breaks). In addition, due to the high concentrations of $\beta$-mercaptoethanol, it appeared to be difficult to control the pH during the alkali-treatment.

Additional studies were directed to conditions to release the DNA from the sperm cells in the alkaline solution itself, without pretreatment, but with simultaneous unwinding. To this end, a number of experiments were carried out in which the concentration of the various substances in the lysis/unwinding solution were varied. This resulted in the following procedure: A straw with frozen bovine sperm was thawed at 37°C and emptied in a 10-ml conical tube and subsequently the sperm was divided over two conical tubes. One of these tubes was irradiated with a dose of 10 Gy $^{60}$Co-$\gamma$-rays at 30°C.

Sperm (10 $\mu$l, ca 5 x $10^5$ cells) was brought into an 8-ml polystyrene roundbottom tube and placed on ice. Then 200 $\mu$l of solution B was added (0.8 M Urea + 100 mM $\beta$-mercaptoethanol + 0.2% Sarkosyl + 1 M NaCl + 0.11-0.13 M NaOH). The sample was transferred to a water bath of 20°C and after 15 min placed back into the icewater bath. Then the solution was neutralized with 100 $\mu$l 0.25 M NaH$_2$PO$_4$ and

sonicated for 5 sec in order to fragment the DNA and finally diluted with 0.9 ml PBS containing 0.05% Sarkosyl.

In an alternative experiment, the 10 $\mu$l sperm in the roundbottom tube was treated at room temperature with 200 $\mu$l solution C (the same as solution B but containing 0.13 M instead of 1 M NaCl). After 15 min the solution was neutralized with 100 $\mu$l 0.25 M NaH$_2$PO$_4$ at 20°C and treated as above. In the DNA samples obtained in this way no intact sperm cells or sperm heads could be observed by microscope. When the treatment with solution C was carried out in the same way (i.e. at 0°C) as with solution B, then intact sperm heads were observed and DNA was insufficiently released. This indicates that the treatment used, both with solution B as well as solution C to release DNA from the heads was rather mild but not optimal. Only freshly prepared solutions of B and C were used to be certain of a reproducible pH (the pH of these solutions appeared to increase slowly; about 0.3 pH unit during 18 h). Also these procedures, however, did not lead to reproducible results.

In another attempt we removed the NaCl completely from solution B and also replaced Sarkosyl with 0.3% Triton X100 (solution D). This appeared to give much better results. Also in the preparations obtained in this way no intact sperm cells or sperm heads were observed microscopically. The presence of Sarkosyl resulted in a precipitate of cell fragments or other material present in the sperm sample. In the presence of 0.3% Triton X100 instead of Sarkosyl, the preparations were microscopically much more clear. Also with Tween 20 (instead of Triton X100) clear preparations were obtained after alkali-treatment. However, the difference in the amount of single strandedness between irradiated and non-irradiated samples was less than with Triton X100. The DNA samples were prepared with various alkaline solutions D differing slightly only in the NaOH concentration (between 0.109 and 0.115 M), because even small differences in the preparation of solution D introduced a large difference in the extent of unwinding. The pH could not be adjusted sufficiently accurate in this pH-range in the presence of the high concentration of $\beta$-mercaptoethanol. The data are summarized in Table 1.

The results indicate that in most cases a dose effect was obtained but the dose-dependence varied still rather strongly. Possibly this was due to small differences in pH in the alkaline solutions used. All these data were obtained with different straws of the same batch of sperm of one sire.

In another experiment we wanted to compare the effect of irradiation on fresh unfrozen sperm and frozen sperm of the same batch. Unfortunately the unfrozen sperm was for practical reasons somewhat more diluted before storage in small aliquots than the frozen samples.

Moreover, sperm preparations of 3 different sires were studied, containing different amounts of cells:

| sire A | fresh:  | ( 3.2x 10$^6$ cells/straw) |
|        | frozen: | ( 6 x 10$^6$ cells/straw) |
| sire B | fresh:  | ( 5 x 10$^6$ cells/straw) |
|        | frozen: | (15 x 10$^6$ cells/straw) |
| sire C | frozen  | (not determined) |

The results are summarized in Table 2. They are clearly different from the expected larger amount of damage in frozen sperm than fresh sperm. In addition, a dose-effect is only observed at higher dose (50 Gy). Furthermore, in frozen sperm a variable (donor-dependent?) amount of single-strand breaks is found. The pH of the alkaline solution after addition to fresh sperm (sire A) appeared to be decreased by 0.23 pH-units, whereas with frozen sperm it amounted to 0.42 units. This strongly suggests that measured differences between fresh and frozen sperm are not due to more damage in fresh sperm, but due to the lower buffer capacity of the fresh sperm, probably the result of the lower concentration of the fresh sperm in comparison to that of the frozen sperm. This may result in a higher pH during unwinding and so a larger unwinding rate. This indicated that the alkali treatment used was not sufficiently reproducible.

Further studies were focussed on alkali-treatment conditions which are less influenced by a) the buffer capacity of the sperm and b) the strong buffer capacity of the thiol-compound. In the literature instead of $\beta$-mercaptoethanol also dithiothreitol (dtt) has been used to release the DNA from the sperm (Hecht and Liem, 1984) and possibly this might be a good substitute for our purposes.

In a treatment of sperm with proteinase K (0.5 mg/ml), dtt (40 mM), 1.2% SDS and 1 mM EDTA in 0.1 M NaCl + 10 mM Tris buffer, pH 8, during 30 min at 65°C, a viscous solution was obtained, which indicated release of DNA.

Pretreatment of sperm with proteinase K with or without dtt, urea and Triton X100 appeared to result in either a premature release of DNA or insufficient release of DNA during the following alkali treatment. This could not be improved effectively with modifications in temperature and period of treatment.

The best results were obtained when, simultaneously with the alkali solution, dtt, urea and Triton X100 were added to a diluted suspension of sperm cells. The 40-fold predilution of the sperm in PBS resulted in an almost complete elimination of the intrinsic buffer capacity of the sperm. Remarkable was the small dose-effect observed, particularly when to the alkali solution also 1.3 M NaCl was added. Only at very high radiation doses some increase of the percentage of single-stranded DNA could be observed. This suggested that already in non-irradiated (frozen) sperm many single-strand breaks are present, or that they are induced during the treatment itself.

To investigate the latter possibility we exposed human white blood cells (WBC), after irradiation of human blood, to the same alkaline conditions as the sperm. In Figure 1 a few dose-effect curves have been presented for WBC treated with different alkaline solutions. The normal alkaline conditions to release DNA from WBC and to unwind it consist of alkali in 1.3 M NaCl, pH 12.1-12.2. Under these circumstances a clear dose-effect can be observed at low radiation doses. In order to be able to measure DNA damage after exposure to higher radiation doses the salt has to be omitted from the alkaline solution. In that case we see, in contrast to that observed with sperm, almost no single-stranded DNA in samples with non-irradiated WBC. So, in spite of the fact that release of DNA from WBC is much easier than from sperm, there is less single-stranded DNA present than in sperm. This suggests that the treatment itself does not induce extra single-strand breaks. Only at a pH far above the pH at which unwinding of DNA can occur, an increase of the number of single-strand breaks in DNA of non-irradiated WBC was observed, but even in that case a dose-effect after doses of 5 and 10 Gy is detectable. Apparently, the single-strand breaks detected in DNA of non-irradiated sperm are already present before the alkali-treatment. These single-strand breaks are possibly the result of accumulation of oxidative damage. In previous experiments it has been observed that in the precursor cells of the spermatozoa, the elongated spermatids, almost no repair of radiation-induced single-strand breaks is present (Van Loon *et al.*, 1991). Furthermore, Fraga *et al.* (1991) reported that the level of 8-hydroxy-2'-deoxyguanosine (a typical oxidative damage in DNA) in (human) sperm is extremely high.

In Figure 2 the induction of breaks as a function of radiation dose is presented for frozen sperm of the two sires A and B, measured after 3 somewhat differing alkali treatment conditions. It is evident that the amount of single-strand breaks measured under all 3 alkali-treatment conditions is higher in DNA of sire B than in that of sire A. In relation to this, it is noted that sperm of sire B is much less efficient in fertilization than that of sire A (personal communication, Holland Genetics).

The reason for the differences between the slopes of the curves is not quite clear. Possibly they are due to an uncertainty in the determination of the total amount of single-stranded DNA with the sandwich ELISA. Another possibility is that, due to still occurring differences in the buffering capacity of the diluted sperm, the rate of unwinding varied somewhat for the different sperm samples. It is not likely that small differences in buffer capacity will influence to that extent the efficiency of the induction of single-strand breaks by ionizing radiation. Based on this assumption, we can circumvent the effects of small variations in buffering capacity and uncertainties in the total amount of DNA by determining a complete dose-effect curve of each sample and to express the amount of oxidative damage in the non-irradiated sperm in Gray-equivalents.

In Figure 2 the intersections of the curves with the X-axis indicate the radiation doses corresponding with the observed background levels. With both sperm preparations the intersection with the X-axis shifts to the left with stronger lysis conditions. This might indicate that induction of breaks is induced by the alkali treatment, contrary to that suggested by the experiments with WBC, or that at the weaker lysis conditions unwinding cannot start at each single-strand break due to incomplete lysis. However, because strong lysis conditions are accompanied by high percentages of unwinding, thus leaving only a small window for measuring differences between sperm preparations, we prefer to apply the lowest concentration of urea (0.5 M) that still results in a satisfactory DNA release and unwinding. Lower concentrations of urea (in particular below 0.4 M, such as 0.25 M) resulted in insufficient lysis.

The method appeared to be also applicable, with the same alkali-treatment conditions, on human sperm. In Figure 3 dose-effect curves are presented of sperm of three different donors. Also in these samples high levels of oxidative damage are observed. The measured differences correlate qualitatively with the measured progressive motility of the samples.

*Sandwich ELISA with $\gamma-ray-$irradiated human blood cells*

The sandwich ELISA described herein was used to analyze the induction of irradiation-dependent alkali-enhanced DNA single-strandedness in white blood cells of human blood exposed to various doses of $\gamma$-

rays. It was our aim to study the usefulness of the sandwich ELISA under conditions that were optimal for reproducible, controlled partial unwinding of DNA in alkaline medium and to compare it with that of the earlier developed ELISA (Van Loon *et al.*, 1992) under the same conditions (see below).

Figure 4 (closed symbols) shows the relation between radiation dose and the relative amount of single-strandedness induced during unwinding. Two independent experiments are shown which were slightly different in the conditions of unwinding of the DNA with respect to pH. To be able to compare the data with the ELISA the samples were prepared according to the procedure usually applied for this assay (Van Loon *et al.*, 1992). The open symbols represent the data obtained with the ELISA, the closed ones those of the sandwich ELISA. It is evident that there is complete agreement between the two methods.

One of the big advantages of the sandwich ELISA is the fact that the nucleated cells in the human blood do not have to be purified before alkali treatment. The alkaline solution can directly be added to a certain amount of human blood. We studied the optimal conditions for a reproducible partial unwinding of DNA in alkaline medium.

Figure 5 shows the relation between radiation dose and the relative amount of single-strandedness induced under various conditions during unwinding, which differed with respect to the time of alkali treatment at two different pH-values. The results show that under all conditions tested partial unwinding can be obtained, the extent of which varies substantially, however. After 6 min at pH 12.2 hardly any unwinding is induced, *e.g.* after a dose of 5 Gy about 8% single-strandedness was measured (at pH values lower than 12.0, no unwinding was observed; data not shown). After longer treatment periods the percentage unwinding increased, and reached about 20% after 20 min at pH 12.2. At a higher pH value unwinding proceeded faster; at pH 12.3 after 6 min about 24% and after 10 min 26% single-strandedness was induced after a dose of 5 Gy. At pH values higher than 12.3 unwinding proceeded so fast that no differences could be observed between the irradiated and non-irradiated cells (data not shown).

It should be noted that the percentage of single-strandedness of DNA in non-irradiated cells also increased with the pH and period of treatment, from 1.1% at pH 12.2 after 6 min to 3.6% at pH 12.3 after 10 min. The dose-response relationships are linear and the induction of breaks is still detectable after a dose as low as 0.5 Gy (data not shown) as is the case with the previously described ELISA (Van Loon *et al.*, 1992). However, the ratio of the percentage single-strandedness at a dose of 5 Gy to that of non-irradiated blood amounted to about 9, whereas with the purified white blood cells, after irradiation of the blood only a ratio of about 4 was observed. Apparently, the lysis of the erythrocytes (at $0°C$), necessary to purify the white blood cells, leads also to some damage to the DNA in the white blood cells resulting in a higher background of single-strand breaks in non-irradiated white blood cells. This suggests that with the sandwich ELISA more realistic levels of single-strand breaks in DNA in human blood can be obtained.

In principle a logarithmic expression is required between the percentage of single-strandedness observed and the number of single-strand breaks present, to compensate for the Poisson distribution of breaks in the genome. However, in practice this appeared to be unnecessary. As can be observed in Figure 3, the S.E.M. of the data-points amounts to only 1 to 2% single-stranded DNA. Nevertheless, even at the highest percentage of single-stranded DNA (after a dose of 5 Gy) the data do not significantly deviate from a straight line through the data-points, both when plotted as in Figure 3 and when the percentage double-stranded DNA is plotted semi-logarithmically as a function of the X-ray dose. Therefore, it seems not necessary to prepare a calibration curve to correct for this.

*Modifications in the coating of polystyrene plates with D1B*

In the original experiments the microtiter plates used for the coating with D1B antibodies have a surface with free COOH groups, to which the antibody molecules are attached covalently via coupling with EDC. For the coating, normally the plates are pretreated with 4 mg EDC/ml. We also tried a solution of 2 mg/ml and 8 mg/ml and the plates were further processed as normal. With plates pretreated with 2 mg EDC/ml a slightly higher fluorescence was observed indicating a better binding of single-stranded DNA. This small increase is not a reason to change the protocol.

In addition, we also varied the concentration of D1B-antibodies applied for the coating. A concentration of 5 $\mu$g (instead of 10 $\mu$g) D1B/ml did not result in significantly different fluorescence values. Application of 1 $\mu$g D1B/ml resulted in fluorescence values which were about 40% lower, but did not result in other values for the percentage single-strandedness derived from the fluorescence data. However, since lower fluorescence values will result in larger uncertainties in the percentage single-strandedness, a concentration of 5 $\mu$g D1B/ml is preferable.

In later experiments, when using high-binding microtiter plates (as described in Materials and Methods) it appeared that EDC-pretreatment is not necessary. In these experiments it also appeared that coating with 10 µg D1B/ml gives the largest range over which the fluorescence is proportional to the amount of single-stranded DNA per well.

*Ratio of D1B and alkaline phosphatase (AP) during coupling*

A change of the ratio of the D1B-AP conjugates from the normally applied 0.5 mg D1B/mg AP to 1 or 2 mg D1B/mg AP resulted in a corresponding decrease of the fluorescence when the conjugates were applied in the sandwich ELISA at the standard concentration. This could be compensated by adding a more than proportionally higher amount of D1B-AP conjugate to the wells. A change of the D1B/AP ratio did not have any effect on the percentage single-strandedness derived from the sandwich-ELISA data.

*Conditions during DNA and D1B–AP binding*

Originally, binding of both DNA and D1B-AP conjugate in the sandwich ELISA was performed by incubation for 45 min at 37°C. However, it appeared that the time needed could be shortened by carrying out the incubation under continuous vibration, even at room temperature. Incubation for 5 min at room temperature appeared to be sufficient. This was checked by detecting D1B antibody-binding after transfer of the remaining solution from the wells to those of another D1B-coated microtiter plate.

*Application of other single–stranded DNA specific monoclonal antibodies*

Several other types of monoclonal antibodies of the IgM or IgG subclasses directed against single-stranded DNA that were raised in our laboratory were applied in this assay. However, the affinity of most of those appeared to be not as high as that of the D1B antibodies, or they lost their activity after having been frozen and thawed. It was however confirmed that monoclonal antibodies different from D1B may be suitable.

*Application of the sandwich ELISA at low doses of X–rays*

As described herein, because of the more favourable ratios of the percentage single-strandedness of irradiated blood to that of non-irradiated blood, in principle, the sandwich ELISA can be applied to detect DNA-damage at lower radiation doses. To study this possibility we irradiated human blood with 300-kV X-rays in a dose range of 0 to 1 Gy. In figure 6 a number of dose-response curves are presented of blood samples of which the alkali-treatment conditions varied somewhat. It is evident that a dose of 0.4 Gy is certainly detectable and with sufficient determinations a difference between non-irradiated blood and blood irradiated with a dose of 0.2 Gy will be detectable.

Table 1

| Percentage single-stranded DNA in samples of frozen-thawed sperm treated with alkaline solution D | | | | | | |
|---|---|---|---|---|---|---|
| | [NaOH] (M) | pH (measured) | 0 Gy | 10 Gy | 20 Gy | 50 Gy |
| exp 1 | 0.115 | 12.24 | 3<br>5.1<br>5.2 | 8.5<br>10<br>7.4 | | |
| exp 2 | 0.115 | 12.41 | 6.9<br>8.7<br>6.6<br>6.3 | 12.4<br>16.0<br>8.3<br>14.5 | 10.9<br>13.4<br>11.6<br>13.3 | |
| exp 3 | 0.115 | 12.41 | 8.3<br>8.2<br>7.3 | 10.5<br>18.0<br>16.9 | 12.4<br>17.0<br>17.5 | |
| exp 4 | 0.115 | 12.43 | 17<br>22 | 21<br>28 | 24<br>30 | |
| exp 5 | 0.115 | 12.47 | 25<br>25<br>40 | 32<br>35<br>76 | | |
| exp 6 | 0.114<br>0.109<br>0.110 | 12.40<br>12.35<br>12.35 | 9.6<br>1.8<br>0.8 | 22<br>2.7<br>8.3 | | |
| exp 7 | 0.114<br>0.111<br>0.113 | 12.41<br>12.33<br>12.38 | 14.9<br>1.3<br>17.5 | 21.7<br>2.8<br>28 | | 31<br>4.6<br>26 |

Note: The pH-values mentioned were determined in the alkaline solution without sperm. For each experiment fresh alkaline solutions were prepared. The data mentioned within one experiment are of the same day.

Table 2. Percentage single-stranded DNA in samples of fresh and frozen sperm treated with alkaline solution D

| | [NaOH] (M) | pH (measured) | 0 Gy | 10 Gy | 50 Gy |
|---|---|---|---|---|---|
| | 0.114 | 12.38 | | | |
| sire A | | | | | |
| fresh | | | 30 | 34 | 62 |
| | | | 33 | | 55 |
| frozen | | | 4.4 | 2.5 | 7.7 |
| sire B | | | | | |
| fresh | | | 33 | 33 | 46 |
| | | | 44 | | 55 |
| frozen | | | 10.3 | 9.7 | 11.7 |
| sire C | | | | | |
| frozen | | | 9.1 | 8.3 | 11.9 |
| | 0.115 | 12.41 | | | |
| sire A | | | | | |
| fresh | | | 43 | 50 | 76 |
| frozen | | | 12 | 12 | 16 |
| sire B | | | | | |
| fresh | | | 50 | 46 | 86 |
| frozen | | | 19 | 22 | 24 |
| sire C | | | | | |
| frozen | | | 20 | 20 | |

REFERENCES

Ahnström, G., and Erixon, K, 1973, Radiation-induced strand breakage in DNA from mammalian cells. Strand separation in alkaline solution. International Journal of Radiation Biology, 23, 285-289.

Balhorn, R., B.L. Gledhill and A.J. Wyrobek, 1977, Mouse sperm chromatin chromatin proteins: Quantitative isolation and partial characterization. Biochemistry, 16, 4074-4080.

Ballard, D.W., Lynn, S.P., Gardner, J.F. and Voss Jr., E.W., 1984, Specificity and kinetics defining the interaction between a murine monoclonal autoantibody and DNA, J. Biol. Chem., 259, 3492-3498.

Ballard, D.W. and Voss Jr., E.W., 1985, Base specificity and idiotype of anti-DNA autoantibodies reactive with synthetic nucleic acids, J. Immunol., 135, 3372-3380.

Campbell, A.M., 1984, Monoclonal antibody technology. Laboratory techniques in biochemistry and molecular biology. Eds. R.H. Burdon and P.H. van Knippenberg. Elsevier, Amsterdam, pages 33-65.

Claassen, E and Adler, L.T., 1988, Sequential double immunocytochemical staining for *in situ* identification of an auto-anti-allotype immune response in allotype-suppressed rabbits. The Journal of Histochemistry and Cytochemistry, 36, 1455-1461.

Evenson, D.P., 1986, Flow cytometry of acridine orange stained sperm is a rapid and practical method for monitoring occupational exposure to genotoxicants. In: (Sorsa, M. and Norppa, H. eds.) Monitoring of Occupational Genotoxicants. alan R. Liss, New york, NY, pp. 121-132.

Evenson, D.P. and L. Thomson, 1992, Flow cytometric analysis of boar sperm chromatin structure as related to cryopreservation and fertility. In: Boar semen preservation. II. Proceedings of the second international conference on boar semen preservation (1990, Beltsville, USA). The Reproduction in Domestic Animals, suppl. 1, p. 165-183. Publication No. 2536 from South Dakota State University Experiment Station.

Fraga, C.G., P.A. Motchnik, M.K. Shigenaga, H.J. Helbock, R.A. Jacob and B.N. Ames, 1991, Ascorbic acid protects against endogenous oxidative DNA damage in human sperm. Proc. Natl. Acad. Sci. USA, 88, 11003-11006.

Frankfurt, O.S., 1987, Detection of DNA damage in individual cells by flow cytometric analyses using anti-DNA monoclonal antibody, Exp. Cell Res., 170, 369-380.

Fukada, M., Yoshikawa, K and Miyagawa, Y., 1988, Recovery of DNA fragments specifically bound by monoclonal anti-DNA antibody, J. Immunol. Methods, 110, 137-141.

Hecht, N.B. and H. Liem, 1984, Mitochondrial DNA is synthesized during meiosis and spermiogenesis in the mouse. Experimental Cell Res. 154, 293-298.

Kohn, K.W., and Grimeg-Ewig, R.A, 1973, Alkaline elution analysis, a new approach to the study of DNA single-strand interruptions in cells. Cancer Research, 33, 1849-1853.

Loon, A.A.W.M. van, P.J. den Boer, G.P. van der Schans, P. Mackenbach, J.A. Grootegoed, R.A. Baan and P.H.M. Lohman, 1991a, Immunochemical detection of DNA damage induction and repair at different cellular stages of spermatogenesis of the hamster after *in vitro* or *in vivo* exposure to ionizing radiation. Exp. Cell Res., 193, 303-309.

Loon, A.A.W.M. van, R.H. Groenendijk, G.P. van der Schans, P.H.M. Lohman and R.A. Baan, 1991b, Detection of base damage in DNA in human blood exposed to ionizing radiation at biologically relevant doses. Int. J. Radiat. Biol., 59, 651-660.

Loon, A.A.W.M. van, R.H. Groenendijk, A.J.Timmerman, G.P. van der Schans, P.H.M. Lohman and R.A. Baan, 1992, Quantitative detection of DNA damage after exposure to ionizing radiation by means of an improved immunochemical assay. Mutation Res. 274, 19-27.

McGrath, R.A., and Williams, R.W., 1966, Reconstruction *in vivo* of irradiated Escherichia coli deoxyribonucleic acid; the rejoining of broken pieces. Nature, 212, 534-535.

Olive, P.L, Banáth, J.P. and Durand, R.E, 1990, Heterogeneity in radiation-induced DNA damage and repair in tumour and normal cells measured using the "Comet" assay, Rad. Res., 122, 86-94.

Rydberg, B., 1975, The rate of strand separation in alkali of DNA of irradiated mammalian cells, Rad. Res., 61, 274-287.

Rydberg, B., 1980, Detection of induced DNA strand breaks with improved sensitivity in human cells, Rad. Res. 81, 492-495.

Schans, G.P. van der, H.B. Centen and P.H.M. Lohman. 1982, DNA lesions induced by ionizing radiation. In: Progress in Mutation Research, Vol. 4, Eds. A.T. Natarajan *et al.*, Elsevier Biomedical Press, 285-299.

Schans, G.P. van der, A.A.W.M. van Loon, R.H. Groenendijk and R.A Baan. 1989, Detection of DNA damage in cells exposed to ionizing radiation by use of anti-single-stranded-DNA monoclonal antibody. Int.J. Radiat. Biol. 55, 747-760.

Sutherland, J.C., Lin, B., Monteleone, D.C., Mugavero, J., Sutherland, B.M. and Trunk, J., 1987, Electronic imaging system for direct and rapid quantitation of fluorescence from electrophoretic gels: application to ethidiumbromide-stained DNA, Anal. Biochem., 163, 446-457.

Traincard, F., Chevrier, D., Mazie, J.C. and Guesdon, J.L, 1989, Monoclonal anti-nucleoside antibodies, characterization and application in an enzyme immunoassay of single-stranded DNA, J. Immunol. Methods, 123, 83-91.

**Claims**

1. A method for detecting single-strand breaks in cellular DNA, comprising the steps of incubating a sample comprising cells containing said DNA with an alkaline medium capable of releasing DNA from

16

the cells and at least partially unwinding said DNA, and subjecting the alkaline medium-treated sample to a sandwich immunoassay using a monoclonal antibody directed against single-stranded DNA to determine the amount of single-stranded DNA therein.

2. A method according to claim 1 wherein said monoclonal antibody is D1B, ECACC number 93021026.

3. A method according to claim 1 or 2 wherein the sandwich immunoassay uses the same monoclonal antibody for immobilizing the single-stranded DNA and for labeling the immobilized single-stranded DNA.

4. A method according to anyone of claims 1 to 3 wherein the sandwich immunoassay is a sandwich ELISA using a conjugate of monoclonal antibody and an assayable enzyme, such as alkaline phosphatase, to label the monoclonal antibody-immobilized single-stranded DNA.

5. A method according to anyone of claims 1 to 4 wherein said sample consists of sperm of a vertebrate.

6. A method according to claim 5 wherein said sample consists of sperm of a mammal.

7. A method according to anyone of claims 1 to 6 wherein the alkaline medium capable of releasing DNA from the cells and of at least partially unwinding the DNA has a pH of above about 12 and comprises alkali metal hydroxyde, urea, dithiothreitol and a surfactant.

8. A method according to claim 7 wherein the alkaline medium has a pH of from about 12.05 to about 12.25.

9. A method according to claim 7 or 8 wherein the surfactant comprises a non-ionic surfactant.

10. A method according to claim 9 wherein the non-ionic surfactant comprises Triton X100.

11. A method according to claim 10 wherein the alkaline medium comprises about 0.4-1.0 M urea, about 40-45 mM NaOH, about 14-18 mM dithiothreitol and about 0.03-0.1% Triton X100.

12. A method according to claim 11 wherein the alkaline medium comprises about 0.5 M urea, about 43 mM NaOH, about 16 mM dithiothreitol and about 0.06% Triton X100.

13. A method according to anyone of claims 1 to 12 wherein said incubation with alkaline medium is carried out for about 3-15 minutes.

14. A method according to claim 13 wherein said incubation with alkaline medium is carried out for about 7 minutes.

15. A method according to anyone of claims 1 to 14 wherein the alkaline medium-treated sample is neutralized and sonicated before it is subjected to the sandwich immunoassay.

16. A method according to anyone of claims 1 to 15 wherein the sample is subjected to a treatment capable of inducing single-strand breaks in the cellular DNA before the treated sample is incubated with the alkaline medium.

17. A method according to claim 16 wherein the treatment capable of inducing single-strand breaks in the DNA comprises irradiating the sample.

18. A method according to claim 17 wherein the sample is irradiated with $\gamma$-rays or X-rays.

19. A method according to claim 18 wherein the sample is irradiated with a radiation dose of from about 0.2 Gy to about 2000 Gy.

20. Monoclonal antibody D1B (ECACC No. 93021026) which is directed against single-stranded DNA.

17

## Damage in DNA of human WBC and sperm spontaneous and radiation-induced

FIG.1

Damage in DNA of sperm of 2 bovine sires
spontaneous and radiation-induced

FIG. 2

FIG. 3

FIG. 4

FIG. 5

FIG. 6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | MUTATION RESEARCH vol. 274, 1992, AMSTERDAM NL pages 19 - 27 A.A.W.M.VAN LOON ET AL. * the whole document * | 20 | G01N33/53 C12Q1/68 C12P21/08 |
| Y | | 1 | |
| A | | 2,4,7,8, 13-19 | |
| Y,D | JOURNAL OF IMMUNOLOGICAL METHODS vol. 123, no. 1, 1989, AMSTERDAM NL pages 83 - 91 F.TRAINCARD ET AL. * the whole document * --- | 1 | |
| A | DATABASE WPI Week 9029, Derwent Publications Ltd., London, GB; AN 90-221313 & JP-A-2 150 769 (TOSOH CORP.) 11 June 1990 * abstract * --- | 1,4 | |
| D,A | EXPERIMENTAL CELL RESEARCH vol. 193, 1991, LONDON GB pages 303 - 309 A.A.W.M.VAN LOON ET AL. * the whole document * --- | 1,5,6 | TECHNICAL FIELDS SEARCHED (Int. Cl.5)  G01N C12Q C07H |
| A | EP-A-0 310 251 (MOLECULAR DEVICES CORPORATION) * page 3, line 4 - line 56 * * page 4, line 2 - line 7 * * page 4, line 17 - line 27 * --- -/-- | 1,9,10 | |

The present search report has been drawn up for all claims

| Place of search | Date of completion of the search | Examiner |
|---|---|---|
| BERLIN | 22 NOVEMBER 1993 | DE KOK A.J. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)

European Patent
Office

**EUROPEAN SEARCH REPORT**

Application Number

EP    93 20 1672
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 057 553 (ATOMIC ENERGY OF CANADA LIMITED)<br>* page 5B, last paragraph - page 6, line 16 *<br>* page 7, line 20 - page 9, line 29 * | 7-12 | |

-----

TECHNICAL FIELDS
SEARCHED (Int. Cl.5 )

The present search report has been drawn up for all claims

| Place of search | Date of completion of the search | Examiner |
|---|---|---|
| BERLIN | 22 NOVEMBER 1993 | DE KOK A.J. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)